# EUROPEAN PATENT SPECIFICATION

(11) **EP 2 580 348 B1**
(45) Date of publication and mention of the grant of the patent: **25.04.2018**
(21) Application number: 11726124.8
(22) Date of filing: 14.06.2011
(51) Int. Cl.: C12Q 1/68, G01N 33/50

(54) **METHOD FOR DETERMINATION OF TARGET CELLS OR TISSUE FOR EXTRACTION OF BIOMOLECULES FROM NON-FORMALIN-FIXED BIOLOGICAL SAMPLES**
VERFAHREN ZUR BESTIMMUNG VON ZIELZELLEN ODER -GEWEBE ZUR EXTRAKTION VON BIOMOLEKÜLEN AUS NICHT-FORMALIN-FIXIERTEN BIOLOGISCHEN PROBEN
PROCÉDÉ DE DÉTERMINATION DE CELLULES OU DE TISSU CIBLES POUR L'EXTRACTION DE BIOMOLÉCULES À PARTIR D'ÉCHANTILLONS BIOLOGIQUES NON FIXÉS AU FORMOL

(30) Priority: 14.06.2010 EP 10165797
(43) Date of publication of application: 17.04.2013
(73) Proprietor: Qiagen GmbH, 40724 Hilden (DE)
(72) Inventor: GROELZ, Daniel, 40724 Hilden (DE)
(74) Representative: f & e patent
(86) International application number: PCT/EP2011/059777
(87) International publication number: WO 2011/157678

(56) References cited:
- WO-A1-2005/068610
- WO-A1-2007/045681
- WO-A2-02/055985
- WO-A2-03/031591
- US-A- 5 506 105
- US-A- 5 589 333
- US-A1- 2002 142 340

## Description

The present invention relates to a method for determination of target cells or tissue for isolating or extracting biomolecules from fixed biological samples, the preparation of a sample in a method for extracting, isolating and/or purifying biomolecules from a fixed biological sample as well as to the use of a kit for visualizing target cells or tissue in a fixed biological sample for extracting, isolating and/or purifying biomolecules from said target cells or tissue.

The work leading to this invention has received funding from the European Community's Seventh Framework Programme *(FP7*/*2007-2013)* under *grant agreement* n° 222916.

The biomolecule status of a biological material taken out from its natural environment alters increasingly, the longer the elapsed time between the sample collection and its analysis. In particular the ribonucleic acids (RNA) degrade quickly due to ubiquitous RNases. Also, besides the degradation of nucleic acids, stress genes, for example, are induced and lead to the synthesis of new mRNA molecules that likewise strongly alter the transcription pattern of the sample. Accordingly, in order to retain the gene expression profile, the sample under examination has to be stabilised immediately.

As chemical analyses of proteins and molecular analyses are also increasingly used in areas other than in the field of medical and clinical diagnostics, such as forensics, pharmacy, food analysis, agriculture, environmental analyses as well as in many research areas, the retention of the integrity of the molecular structure of samples and consequently their immediate stabilisation, is therefore a necessary requirement in all these fields.

Thus, over the years, a great number of the most varied stabilisation reagents and stabilisation methods have been developed for the stabilisation of a wide range of the most varied biological materials.

Stabilisation by means of formalin and the subsequent embedding of the stabilised samples for the histological examination of tissue has been known for a long time. However, this type of stabilisation is mostly unsuitable for use in molecular biological processes, as only a very insufficient stabilisation of the biomolecules results, and so at most, a qualitative, but not quantitative indication of the biomolecules, in particular nucleic acids or nucleic acid fragments present is possible. Moreover, the stabilisation with crosslinking stabilisers such as formalin leads to a reduced extractability of the nucleic acids or proteins from the tissue. Formalin is also toxicologically not harmless.

Stabilisation reagents, such as cationic detergents are described in US 5,010,184, US 5,300,545, WO-A-02/00599 and WO-A-02/00600, with which very good qualitative identifications of nucleic acids can be achieved. Other stabilisers that comprise, for example, high concentrations of ammonium sulfate (see e.g. US 6,204,375), are very suitable for stabilising nucleic acids in various tissues.

Within one and the same biological material, very different tissue and/or cell types can be comprised, which differ, for example, in the cell structure, the cell integrity and the expression pattern of the biomolecules.

Tissue fixatives for preservation of morphological structures can be divided into two groups. Group one contains crosslinking agents like formaldehyde, paraformaldehyde or glyoxal. From these 4% buffered formaldehyde is the most widely employed fixative. The only change made since its introduction in 1896 by F. Blum is neutral buffering of formalin at a physiological pH (NBF, neutral buffered formalin). Although not fully understood it is thought that the aldehydes form cross-links between proteins. By this enzymatic activity is ceased and soluble proteins are fixed to structural proteins. Since formaldehyde also reacts with nucleic acids, NBF fixation leads to low yield, degradation of nucleic acids and inhibition in downstream reactions like PCR (polymerase chain reaction) or RT- (reverse transcription) PCR.

The second group does not contain crosslinking agents, but alcohols as major components. Noncrosslinking, alcoholic fixatives were introduced to avoid hazardous formaldehyde fixation and to better preserve tissue macromolecules and especially protein epitops for immunohistochemical methods. Alcohols have to penetrate the tissue and to reach a certain local concentration for precipitating and denaturation of proteins. It has been found that some of the alcoholic fixatives like Carnoy's (60% ethanol, 30% chloroform, 10% acetic acid) or Methacarn (Carnoy's with the substitution of ethanol with methanol) yield superior results as nucleic acids fixatives compared to aldehydes (Cox et al., Experimental and Molecular Pathology 2006; 80: 183-191). Other published fixatives based on alcohol consist of 70% ethanol (Gillespie et al., Am. J. Pathol. 2002; 160(2):449-457), 56% Ethanol and 20% PEG (polyethyleneglycol, Bostwick et al.; Arch.Pathol. Lab. Med. 1994; 118:298-302) or 90% methanol and 10% PEG300 (Vinvek et al., Lab. Investigation 2003; 83(10): 1427-35).

Furthermore several patent applications describe improved noncrosslinking fixatives. In DE 199 28820 a fixative is disclosed containing a mixture of different alcohols, aceton, PEG and acetic acid. In US 2003/0211452 A1 a fixative commercialised as "Umfix" is described containing at least 80% methanol and up to 20% PEG300 for preservation of RNA, DNA and morphology. Fixatives based on ethanol for preservation of molecular content and morphology are described in US 2005/0074422 A1 ("Finefix"), WO 2004/083369 ("RCL2") and WO 05/121747 A1 ("Boonfix"). In US 2003/119049 an universal collection medium is described which is water based and comprises a buffer component, one alcohol, a fixative component and an agent to inhibit degradation.

Beside crosslinking and alcoholic fixatives there are a number of agents known which combine both groups of fixatives. One example is "Genofix", described in US 5,976,829 A for preservation of morphology, immunogenicity as well as RNA and DNA integrity. Since formaldehyde is one component "Genofix" also shows all the disadvantages of crosslinking agents.

EP-A 1 965 190 describes a concept of fixation of a biological sample wherein the sample is fixed by contacting the sample first with a non-aqueous composition comprising up to 90% methanol and an additional additive and thereafter contacting the sample with a second composition comprising up to 99% ethanol. Using this method a very good stabilisation of the biomolecules contained in the biological sample is obtained.

Isolation of mRNA from stained, formalin-fixed samples is known, e.g. described in WO 2007/045681 A1, however, the state of the art doesn't provide an effective method for isolating biomolecules from non-formalin-fixed biological samples.

The object of the present application was to provide a method for extracting and isolating biomolecules from non-formalin-fixed biological samples, wherein the obtainable biomolecules are representing the biomolecules pattern of cells or tissue of interest (" target cells") of said sample.

This object is met by a method for determination and/or selection of target cells or tissue for isolation, extraction or purification of biomolecules from a fixed biological sample, comprising the steps of:
(1) mounting at least one part of the fixed biological sample on at least one support,
(2) optionally treating at least one part of the sample with a sample treatment agent, wherein said treatment can be carried out at any stage of the procedure before step (6)
(3) staining at least one part of the fixed biological sample mounted on at least one support for not more than 60 seconds with at least one cell or tissue staining agent and rinsing the sample with water,
(4) visualizing or considering under light or backlight the at least one part stained in step 3 to determine target cells or tissue comprising biomolecules of interest,
(5) separating the target cells or tissue from the remainder of the fixed sample mounted on the support,
(6) isolating the biomolecules from the separated cells or tissue. wherein the biomolecules comprise RNA;
wherein the fixed biological sample is a non-formalin-fixed sample

Preferably according to the invention the steps are carried out in the provided order, wherein step (2) is carried out at any stage after step (1) and before step (6). Preferably step (2) is carried out before step (3). Steps (1) to (5), in particular steps (3) to (5) serve predominantly to allow - preferably by visual consideration of the stained sample - determination and/or selection of cells probably including biomolecules of interest, which are then isolated according to step (6). It is particularly preferred according to the invention that the determination / selection of target cells is carried out before the biomolecules are isolated.

The biological sample may be a whole organism, a part of an organism, in particular a tissue fragment or a tissue section or cultivated cells or tissue, originating from humans, animals or plants, or microorganisms. Embedded cells isolated from cell cultures may be used as well. Suitable biological samples are all biological samples suitable for fixation, such as, for example, cell-containing bodily fluids such as blood, sperm, cerebrospinal fluid, saliva, sputum or urine, leukocyte fractions, buffy coats, faeces, surface biopsies, aspirates, skin fragments, entire organisms, organs and tissue of Metazoa, preferably of insects and mammals, in particular of humans, for example in the form of autopsies, biopsies, fine-needle aspirates or tissue sections, isolated cells, for example in the form of adherent or suspended cell cultures, plants, parts of plants, plant tissue or plant cells, bacteria, viruses, yeast and fungi.

The term "fixing", "fixation" or "fixed" means that the biological sample is treated with an agent or a composition resulting in a stabilisation of the morphological and/or histological structure, particularly in a stabilisation of the biomolecules contained in the sample. Examples for such agents or compositions known from the state of the art are described above. According to the invention the biological sample was fixed with a composition not comprising formalin, which according to the present invention is described as "non-formalin-fixed sample". Preferably the biological sample was fixed with commercially available noncrosslinking compositions as described above, like "Carnoy's", "Methacarn", "Umfix", "Finefix", "RCL2"," Boonfix" or the fixative as described in DE 199 28820 A1. In a particularly preferred embodiment the method of the invention is carried out with biological samples which are fixed by the commercially available "PAXgene® Tissue" Kit (PreAnalytix GmbH, Hombrechtikon, Switzerland), according to the procedure described in EP-A 1 965 190. By means of fixation it is intended to substantially preserve the biological structures in a life-like fashion, so that "real assessment" is possible. A further advantage of fixation is the fact that the specimens can be stored as documents for a long time. Furthermore, many morphologically investigations are only possible on the basis of fixed material.

The term "biomolecules" comprises any type of nucleic acids and proteins of the biological sample. The preferred biomolecules according to the invention are nucleic acids. For said biomolecules, "stabilisation" is intended to mean preferably the inhibition of the degradation, the modification or the induction of the biomolecules. In the case of histological analyses of biological materials, the term "stabilisation" is intended to mean preferably the prevention of a significant change of the morphology of the samples.

"Nucleic acids" according to the present method includes all nucleic acids known to the person skilled in the art, for example natural or synthetic nucleic acids, and also nucleic acids artificially introduced into the sample, single- and doublestranded nucleic acids, straight-chain, branched or circular nucleic acids, in particular DNA and RNA of any type, preferably RNA, in particular mRNA, tRNA, hnRNA, siRNA, miRNA, snRNA or ribozyms, and DNA, in particular genomic or plastidic DNA or DNA from organelles, and also nucleic acids of infectious origin. Preferably according to the present invention the nucleic acids comprise RNA of at least one of the before mentioned type. From a nucleic acid containing sample stabilized good enough to isolate RNA from it usually it is as well possible to isolate DNA, since DNA is compared to RNA more stable and accordingly less susceptible to degradation.

The term "protein" means any protein or peptide, glycosylated or non-glycosylated, naturally occurring or artificially included in the biological sample, like e.g. structural proteins, receptor proteins, enzymes, soluble proteins of the cytoplasm, membrane proteins, antibodies, hormones or any further protein.

For the present invention it is not necessary, however, for long term storage it is very common that the fixation of the biological samples is followed by a step of embedding the fixed material in a wax, usually paraffin (so-called "fixed, paraffin-embedded" (FPE) material). The main purpose of the embedding medium is to permit the specimens to be sectioned and mounted in the natural state for microscopic and/or histochemical applications. For many applications it is however necessary or at least advantageous to remove the embedding medium from the sample, for example prior to histological staining of the sample.

For the present invention any of the known embedding materials can be used, particularly any wax, agar, ester or polyester wax, resins like epoxy resins or nitrocellulose as well as double embedding methods with agar-paraffin, agar-ester wax or nitrocellulose-paraffin wax. Such a wax usually consists of a complex mixture of higher hydrocarbons and may include further components such as esters of higher fatty acids and/or glycols and the like. The wax may be of natural and/or synthetic origin like polyester wax (mixture of polyethylene glycol distearate and 1-hexadecanol) and may additionally contain additives enhancing its sample-embedding properties such as for example DMSO or higher polyolefins. Preferably, the wax may represent paraffin, being a mixture of primarily saturated hydrocarbons solid at room temperature, which typically is prepared by distillation of petroleum. Optionally the paraffin wax may contain any additives.

According to step (1) of the present method at least one part of the fixed biological sample is mounted on at least one support. It is preferred that the biological sample is mounted on a transparent support, like e.g. made of glass or any transparent polymer. Such a support is for example a glass plate like a microscope slide, a plate made from Plexiglas or a clear polymer (e.g. PET), a cell cultivating dish or the like. Of course several parts or sections of the sample can be mounted on one support or several sections or parts can be mounted on several supports.

The fixed and optionally embedded biological sample preferably is sectioned in portions having a thickness of 0,5 to 12 µm, if the sample has not the according thickness or less themselves, like for example cells of a cell culture. It is particularly preferred that the sample is sliced or cut in sections which are translucent enough that they can be considered in detail under light or backlight, e.g. under a microscope. Preferably the thickness is from 1 to 10 µm, more preferred from 2 to 8 µm and most preferred from 4 to 6 µm.

If in the present invention a wax-embedded sample is used said sample preferably is treated according to step (2) with a sample treatment agent. Said sample treatment agent preferably is a de-waxing agent or composition, more preferably a de-paraffinizing agent or composition. Traditionally, de-waxing or deparaffinization involves the use of aromatic solvents such as toluene and, in particular, xylene. On the other hand non-toxic agents or compositions can be used comprising at least one organic solvent / compound immiscible with water. The wax-solubilizing agent may be liquid, at least in the moment of contacting it with the sample. Preferably said solvent may be selected from the group comprising linear, branched and cyclic C₆-C₁₆ alkanes, linear, branched and cyclic dialkyl ethers, wherein each alkyl chain comprises of from 6 to 16 carbon atoms, and oils liquid at room temperature (23 ± 2°C), more preferably selected from the group comprising C₇-C₁₆ alkanes, dialkyl ethers of the general formula O(CₙH₂ₙ₊₁)₂, wherein n is an integer of from 8 to 12, mineral oil, silicon oil and paraffin with a melting point below room temperature, preferably below 15 °C, and most preferably selected from the group comprising xylene or commercially available xylene substitutes like e.g. Shandon xylene substitute (Thermo Fisher Scientific,Waltham, USA), heptane, hexadecane, dioctylether, or mixtures thereof. The melting point of any solvent applied to the wax-embedded sample either in a pure form or as a mixture of solvents, respectively may be below 25 °C, preferably below room temperature. Herein, the term "pure form" refers to a solvent applied to the wax-embedded sample in order to dissolve the wax without being diluted previously and/or mixed with other solvents. It does however not refer to a particular purity grade with respect to the presence of further compounds which do not represent a solvent. If a solvent is applied to the sample in a pure form it preferably has a melting point below room temperature. "Solvents" having a melting point above room temperature may, however, be applied to the wax-embedded sample, if they are present in a mixture of solvents, which is liquid at room temperature. All of the above solvents are able to dissolve the solid paraffins within a few seconds or at most a few minutes (preferably of from about 15 seconds to about 15 minutes) at room temperature.

Typically, the support-mounted sample is immersed in a bath containing the de-waxing or deparaffinizing agent or composition, like e.g. a xylene bath until the paraffin is solubilized. In subsequent steps the deparaffinized sample may be washed by a series of alcohol solutions of decreasing alcohol concentration to remove de de-waxing agent prior to a final wash using water, in order to make the sample accessible for aqueous reactant / reagent solutions, such as for example staining solutions or lysis buffers.

It is preferred that at least one part of the fixed embedded biological sample is stained according to step (3) during the method of the present invention and therefore is de-waxed before staining. If on the other hand a part or section of the sample is not stained before biomolecules are isolated, de-waxing of that part of the sample can be carried out after any of steps (3), (4) or (5) of the method.

Staining of at least one part or section of the sample can be carried out by any known histological staining method, in case that the stained section or part is not used for biomolecule isolation, but as a reference for the section or part used for biomolecule isolation. On the other hand, if the stained section shall be used thereafter for biomolecule isolation it is particularly preferred to use the staining method described below as "quick staining method" to minimize particularly RNA degradation.

According to a standard staining method the samples mounted on the support after de-waxing and rinsing with alcohol solutions and water are incubated in staining solutions according to the instructions of the supplier of the staining agents. An example for a standard staining method is staining the sample for 1 to 3 min in a first staining agent like e.g. Hematoxylin (Merck), rinsing the sample thereafter for 3 to 5 min with running tap water, staining the sample for 1 to 2 min in a second staining solution like e.g. Eosin (Merck), rinsing the sample for 3 to 5 min with running tap water, contacting the stained sample for 1 min with 80% ethanol, for 2 min with 96% ethanol, for 3 min with 100% ethanol and 5 min with xylene. A sample stained by this or any comparable method can be used as a reference for a sample used for biomolecule isolation as described below.

According to a new developed "quick staining method" the sample, optionally after de-waxing and rinsing in alcohol solutions and short incubation in water (preferably only for a few seconds, e.g. 5 sec or only 1 sec), is stained for not more than 60 sec, preferably less than 45 sec, more preferably less than 30 sec, most preferably less than 15 sec with at least one cell or tissue staining agent, rinsing the sample with water, (preferably only for a few seconds, e.g. 5 sec or only 1 sec) and thereafter optionally contacting the sample for not more than 60 sec, preferably less than 45 sec, most preferably less than 10 sec with a further cell or tissue staining agent.

Preferably in this method the first staining agent is contacted with the sample up to 15 sec and the second staining agent is contacted with the sample up to 3 sec, e.g. 1 sec. Rinsing of the sample with water is carried out preferably by "dipping" the sample into water for 1 to 2 sec. Examples for the staining agents are Hematoxylin (Merck) (7,11b-dihydroindeno [2,1-c] chromene-3,4, 6a,9,10(6H)-pentol, C₁₆H₁₄O₆) for the first staining agent and Eosin (Merck) for the second staining agent. Further suitable staining agents are Toluidinblue, Azan, PAS (Periodic acid Schiff staining), however, the staining with Hematoxylin/Eosin is preferred. (Eosin Y: 2',4',5',7'-tetrabromo-3',6'-dihydroxyspiro [2-benzofurane-3,9'-xanthene](-1-on-bisodium salt. C₂₀H₆Br₄Na₂O₅)

In a preferred embodiment the fixed sample is a wax-embedded sample fixed with a composition not comprising formalin ("non-formalin-fixed paraffin-embedded" non-FFPE sample), thus the "quick method" preferably comprises further a step of de-waxing the sample mounted on the support and rinsing the sample after de-waxing with a series of compositions comprising decreasing amounts of alcohol before staining.

If the sample stained by this new method shall be used for RNA isolation it is preferred that for the preparation of all the compositions (staining compositions, alcohol containing compositions) and for the rinsing steps RNase free water is used.

By using this quick staining method it is possible to extract / isolate biomolecules, particularly nucleic acids from the stained sample (section). In particular it is possible to select cells or tissue regions of interest for isolation of nucleic acids on basis of the staining and to isolate then the nucleic acids from said selected cells or regions.

In step (4) of the present method at least one part of the sample which was stained according to step (3) is considered in detail, preferably under light or backlight, e.g. under a microscope. By considering the stained sample e.g. visually it is possible to distinguish and identify different tissue or cell types and to determine whether the sample comprises cells or tissue regions of particular interest, e.g. tumor cells, cells of tissue compartments of interest, foreign tissue or similar. Such cells or tissue serve as target cells or tissue for biomolecule isolation.

By the method of the present invention two particular embodiments can be carried out. Either the biomolecules are isolated from an unstained sample, for which a stained sample serves as a reference. Or the biomolecules are isolated from a stained sample, then preferably the quick staining method should be used.

In the first case it is preferred that the method is carried out by the following procedure:
The fixed biological sample is divided in sections (slices) of 0,5 to 12 µm thickness,
in step (1) at least two of said sections which were in the original sample adjacent to each other are mounted on separate transparent supports each,
optionally at any stage of the procedure independently from each other the sections are treated with a de-waxing agent according to step (2) in case that a wax-embedded sample was used,
in step (3) at least one of the sections is stained with at least one tissue staining agent and an adjacent section is not stained,
in step (4) cells or tissue of interest is/are determined and marked in the stained sample section and the transparent support with the unstained sample section is stacked on top of the stained and marked sample section in the same orientation as in the original sample,
in step (5) the cells or tissue of the unstained sample on top of the marked area of the stained sample is/are separated from the remainder of the unstained sample, the remainder is waived an the cells/tissue corresponding to the marked area of the stained sample is retained,
in step (6) nucleic acids are isolated or extracted from the cells retained in step (5).

In the latter case it is preferred that the method is carried out according to the following procedure:
The fixed biological sample is divided in sections (slices) of 0,5 to 12 µm thickness,
in step (1) at least one of said sections is mounted on a transparent support,
in step (2) the section is treated with a de-waxing agent in case that a wax-embedded sample was used,
in step (3) said section is stained with at least one tissue staining agent according to the "quick staining" method as described above,
in step (4) cells or tissue of interest is/are determined in the stained sample section,
in step (5) the cells or tissue of interest is/are separated from the remainder of the stained sample, the remainder is waived,
in step (6) nucleic acids are isolated or extracted from the cells obtained in step (5).

Thus, consideration of the target cells or tissue either is made in the sample further used for isolation of biomolecules, or is made in a reference sample (an adjacent or corresponding sample), wherein the cells or tissue of interest can be determined and in a non-stained sample the corresponding cells can be found and separated from the remainder of the cells or tissue. Biomolecules are then isolated from the separated target cells or tissue.

The separation of the cells according to step (5) can be carried out by any known method of the state of the art, like scraping away the non-selected cells or tissue, cutting out the target cells or tissue, picking the target cells, sucking the target cells, microdissection, laser dissection (like laser captured microdissection) or any similar method. The preferred method for separating the target cells or tissue is the microdissection of the target cells or tissue and scraping or wiping away the non-selected cells or tissue.

The step of extracting / isolating the biomolecules from the separated selected cells or tissue may be carried out by any of the known biomolecule isolating methods. The used isolation method is not limiting the invention, as long as an effective method is used allowing the isolation of said biomolecules in an effective way. However, it is preferred to use an isolating method suitable for isolation of nucleic acids from small samples.

In a first step of the isolation method a solution, preferably an aqueous solution for lysing the selected cells or tissue is added to the cells or tissue. As a lysis solution any solution, preferably any aqueous solution may be used which is able to lyse/destroy the cells in a cell-containing material, thus releasing the biomolecules into solution. Many aqueous lysis or digestion buffers are known from the state of the art which can be used in the method of the present invention. If the selected part of the sample is still mounted on the support (e.g. the non-selected parts were scraped away after a microdissection), the lysis solution may be brought in contact with the target cells or tissue directly on the support, e.g. by pipetting the solution on top of the target sample. If the target cells or tissue was detached from the support, e.g. by picking, sucking or scraping, the lysis solution can be brought in contact with said target sample part e.g. in a cup or tube.

After contacting the target cells or tissue with the lysis solution the mixture may be homogenized, e.g. by pipetting up and down, by vortexing or any other suitable method. If the target sample part was contacted with the lysis solution on top of the support, the whole mixture of target sample part and lysis solution after an incubation preferably is transferred to a suitable container for further processing, like e.g. a cup or a tube.

Which of the known lysis solutions is appropriate for lysing the target cells or tissue depends mainly from the cells to be lysed. The used lysis solution can be for example a choatropic salt containing lysis buffer, a surfactant containing lysis buffer, a lysis buffer comprising a proteolytic compound or a lysis buffer comprising several or a mixture of said compounds, without being restricted to these.

The method of the present invention can be carried out with a wide range of lysis solutions and lysis protocols known from the state of the art. The lysis solution may for example comprise a buffering agent, preferably selected from the group comprising Tris, Mops, Mes, Hepes and Citrate, optionally a detergent, preferably selected from the group comprising nonionic, cationic, anionic and zwitterionic detergents, more preferably anionic detergents, most preferably selected from the group comprising sodium dodecyl sulfate. Nonionic detergents can be, for example ethylene oxide condensation products, such as ethoxylated fatty acid esters of polyhydric alcohols. A preferred nonionic detergent is a polyoxy ethylene sorbitan monolaurate sold under the trade name TWEEN® 20 by Sigma Chemical Co. Other TWEEN® detergents are also suitable. Other suitable detergents can be taken from the group of Triton detergents (octylphenoxypolyethoxyethanol), advantageously Triton X-100 (also available from Sigma). The detergents are included in an effective amount to lyse the cells, wherein ranges of 1.5 - 20% (v/v) typically being effective. The detergents may also form micelles and other complexes with the nucleic acids, thereby protecting DNA via other mechanisms.

The lysis solution may comprise one or more additional substances, preferably selected from the group comprising chelating agents, reducing agents, inorganic salts and preferably may have a pH in the range of from 5 to 11, preferably of from 6 to 8, and most preferably of from 6 to 7.

In addition to a lysis solution a proteolytic agent may be added to the mixture of the lysis solution and the target cells or tissue. The proteolytic agent may already be comprised in the lysis solution added to the sample or may e added after contacting the cells or tissue with the lysis solution. Said proteolytic agent may preferably be selected from the group comprising proteases and non-enzymatic proteolytic compounds, preferably may represent proteinase K, trypsin, chymotrypsin, papain, pepsin, pronase, endoproteinase Lys-C, bromocyane or a mixture thereof.

The step of incubating the mixture obtained after adding the lysis solution to the sample may preferably be carried out at a temperature in the range of from 2 to 95 °C, preferably of from 15 to 70 °C, and most preferably of from room temperature to 60 °C. This incubation may preferably be carried out for 10 seconds to 10 hours, more preferably for 45 seconds to 5 hours, and most preferably for 1 minute to 2 hours. However, both, temperature and time of incubation may vary with the kind, amount and age of the sample, and the lysis solution employed. This is well known to a person skilled in the art, and optimum lysis conditions, in particular in respect of incubation time and temperature can be easily determined by a person skilled in the art by means of routine experiments.

The step of isolating and/or purifying the biomolecule released from the target cells or tissue and then comprised in the lysing mixture may preferably be carried out by means of any of the known methods of the state of the art.

Preferably the nucleic acids are isolated by at least one chromatographic and/or solid phase-based method, preferably selected from the group comprising a chaotrope or non-chaotrope mediated adsorption to silica beads or membranes, gel filtration chromatography, ion exchange chromatography, reversed phase chromatography and chaotrope-free affinity binding (adsorption).

Several commercially available kits for the isolation of biomolecules, including DNA and RNA, are available on the market. Furthermore several kits for isolation nucleic acids from fixed biological samples have been developed, most of them comprising a deparaffinization agent, a lysis buffer and further buffers and equipment for extracting, isolating and purifying the nucleic acids. Examples for such commercially available kits are PAXgene® Tissue kits from PreAnalytix (Hombrechticon, Switzerland), RNeasy, AllPrep, DNeasy and QIAamp kits from QIAGEN (Hilden, Germany), High Pure Kits from Roche Applied Science (Mannheim, Germany), PureLink Kits from Invitrogen (Carlsbad, USA), RecoverAll Kits from Applied Biosystems (Foster City, USA).

In a particularly preferred embodiment of the invention for fixing the sample, optionally de-waxing the sample and nucleic acid isolation the compounds and compositions of the PAXgene Tissue Container in combination with the PAXgene® Tissue RNA, miRNA or DNA kit from PreAnalytix can be used, the staining of the sample (parts) is carried out with Hematoxylin and Eosin and for RNA isolation the sample part is stained by the quick staining method described herein. It is particularly pointed out that DNA can be isolated with good results from samples from which only less degraded RNA is isolatable, since DNA is more stable than RNA.

According to the present invention as well the use of a kit is provided for visualizing biomolecules comprising target cells or tissue in a fixed biological sample during a method for extracting, isolating and/or purifying biomolecules from said target cells or tissue comprising
a. optionally at least one transparent support
b. at least one tissue staining agent
c. at least one sample treatment agent as described above
d. optionally instructions for a method according to claim 7, 8 or 9
e. at least one buffer and/ or equipment for isolating biomolecules, comprising RNA.

In a preferred embodiment the kit comprises at least one, preferably at least two, more preferred all three of the components (a), (b) and (c) in combination with components (d) and (e).

### Figures:

Fig. 1: RNA yield from decreasing areas of 6µm sections mounted on a slide according to Example 1. Reference: one section was directly transferred into a microcentrifuge tube; on slide: different areas of the original section (50, 25 and 10%) were used for RNA extraction; all extractions were done in triplicates.
Fig.2: RNA Integrity of RNA isolated from decreasing areas of 6µm sections mounted on a slide and the reference according to Example 1. Integrity measured as RIN - RNA Integrity Number.
Fig.3: Performance of RNA isolated according to Example 1 in quantitative RT-PCR. 1 µl of eluate was used as template for a one step RT-PCR using β-actin primer/probe assay on a TaqMan 7400.
Fig. 4: RNA yield from one 4µm section of paraffin embedded tissue according to Example 2. Reference: section transferred into microcentrifuge tube for RNA isolation; staining 1 and 2: RNA extraction from the slides after quick-staining; all extractions done in triplicate.

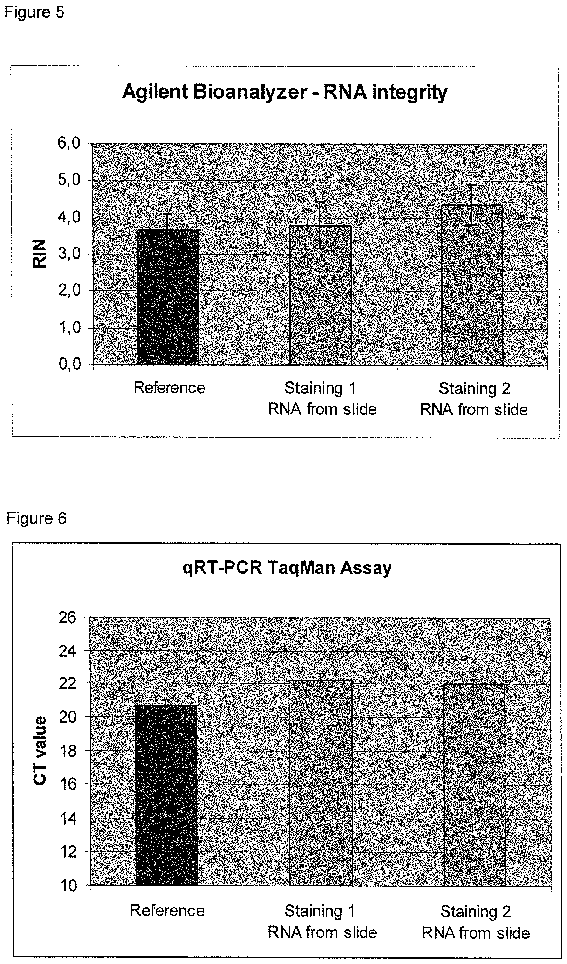

Fig.5: RNA Integrity of the RNA isolated according to Example 2, measured as RIN value using the Agilent Bioanalyzer. RNA was isolated from one 4 µm section of paraffin embedded tissue. Reference: section transferred into microcentrifuge tube for RNA isolation; staining 1 and 2: RNA extraction from the slides after quick-staining; all extractions done in triplicate.
Fig.6: Performance of RNA isolated according to Example 2 in quantitative real time RT-PCR. 2 µl eluate was used as template for one step qRT-PCR using
β-actin primer/probe assay on a TaqMan 7400. RNA was isolated from one 4 µm section of paraffin embedded tissue. Reference: section transferred into microcentrifuge tube for RNA isolation; staining 1 and 2: RNA extraction from the slides after quick-staining; all extractions done in triplicate.

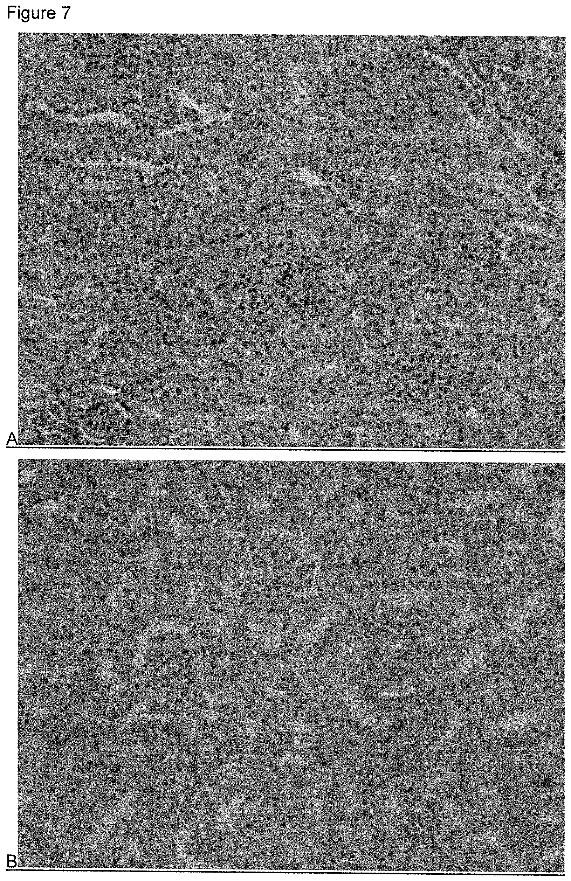

Fig.7: Hematoxylin and eosin quick staining of rat kidney tissue according to Example 2. A: Staining 1, B: Staining 2

### Examples

### Experimental procedures:

### Tissue Fixation:

Fixation by PAXgene® Tissue Fix (PreAnalytix): a considered tissue specimen was submerged into 5 ml of fixation reagent. Incubation was performed for 2-4 hours at room temperature followed by a transfer into PAXgene® Tissue Stabilizer solution.

Fixation by Formalin: tissue was submerged into 5 ml of neutral buffered formalin kept at room temperature and fixed for 24 hours at room temperature.

### Tissue processing and paraffin embedding:

Fixed tissue specimens were transferred from the fixation solution to a standard histocasette. Processing was performed on a Leica tissue processor TP1020, using a protocol as recommended in the PAXgene® Tissue Container handbook, for preservation of biomolecules (dehydration with 15 min 70%, 30 min 80%, 60 min 90% and two times 99% ethanol, two times 60 min 100% Isopropanol, two times 60 min 100% Xylene followed by 60 min Xylene-Paraffin (50-50%) at 50 C and two times 60 min Paraffin at 56 °C). As paraffin, low melting Paraplast-XTRA (SPI supplies) was used. After processing tissue was embedded into a paraffin block, using the same paraffin as for the incubation.

### Hematoxylin and Eosin staining of tissue mounted on glass slides:

Hematoxylin and eosin staining was performed manually by incubation of 4-6 µm sections mounted on slides in xylene for 5 min, followed by 96%, 80%, 70%, 60% ethanol for 3 min each, 3 min incubation in running tap-water, 1 min incubation in Hematoxylin (Merck, ready to use), 3 min running tap-water, 1 min incubation in Eosin (Merck, ready to use), 3 min running tap-water, 1 min 80%, 2 min 96%, 3 min 100% ethanol and 5 min 1005 xylene. Finally the sections were overlaid with entellan (Merck) and a cover slip.

### Modified, quick staining method using Hematoxylin and Eosin for staining tissue mounted on glass slides:

Hematoxylin and eosin staining was performed manually by incubation of 4-6 µm sections mounted on slides in xylene for 5 min, followed by 96%, 80%, 70% ethanol for 1-3 min each, 2-10 sec incubation in RNase free water, 30 sec incubation in Hematoxylin (prepared according to Gill), tapping for 1sec in RNase free water, 1 sec incubation in Eosin (0.5% in RNase free water), tapping 1 sec in RNase free water, 1 min 80%, 2 min 96%, 3 min 100% ethanol and 5 min xylene. For microscopy only the sections were further incubated 5 min in xylene, overlaid with Entellan (Merck) and sealed with a cover slip.

### Preparation of fresh staining solutions:

### Hematoxylin solution according to Gill

2 g Hematoxylin, 0.2 g sodium iodate, and 17.6 g aluminum sulfate (·18H₂O) were dissolved in a mixture of 250 g ethylene glycol and 730 ml RNase free water. 20 ml glacial acetic acid was added, and stirred for 2 hours at room temperature.

### Hematoxylin solution according to Harris

100 g potassium alaun were dissolved in 950 ml RNase free water by stirring and heating. 5 g Hematoxylin were dissolved in 50 ml ethanol under heating in a waterbath. The Hematoxylin solution was added to the hot alaun solution and boiled. After removing from heat 370 mg sodium iodate were added. 4 ml glacial acetic acid were added and the obtained solution was filtered before use.

### Eosin Y solution 0.5% aqueous

5 g Eosin Y were added to 1000 ml RNase free water and stirred for 2 hours at room temperature. 2 ml glacial acetic acid were added while stirring.

### DEPC-treated water

0.1 ml DEPC (diethyl pyrocarbonate) were added per 100 ml of water. To bring the DEPC into solution it was vigorously shaken, incubated for 12 hours at 37°C and autoclaved for 15 minutes to reduce residual DEPC.

### RNA extraction from reference samples:

From sections with 4-6 µm thickness (performed with a Leica RM2245 Microtom) paraffin was removed by incubation in xylene and ethanol as stated in the PAXgene® tissue RNA protocol "Purification of Total RNA from Sections of PAXgene Treated, Paraffin-Embedded Tissue". After removal of xylene and ethanol, the pellet was dissolved with lysis buffer TR1, water and digested for 10 min at 55 °C. Homogenization was performed using the PAXgene® Shredder. After adding ethanol the lysats were transferred onto a PAXgene® MinElute column, washed with buffer TR2, incubated with DNase on the column, washed with TR3 and 80% ethanol according to the protocol. RNA was eluted with 15-40 µl of buffer TR4.

### RNA extraction from whole sections or dissected areas of tissue mounted on a glass slide:

Tissue sections mounted on a glass slide were deparaffinised by incubation for 5 min in xylene and 3 min in 100% ethanol. After staining and/or removal or unwanted areas of the tissue, the selected tissue on the slide was overlaid with 150 µl of a lysis mixture containing protease, water and buffer TR1 (150 µl TR1, 10 µl protease, 290 µl water). After 10 min incubation at room temperature, the mixture and the tissue was transferred by pipetting into a microcentrifuge tube. Residual lysis mixture was added, and incubation 10 min at 55 °C followed. Homogenization was performed using the PAXgene®-Shredder. After adding ethanol the lysats were transferred onto a PAXgene® MinElute column, washed with buffer TR2, incubated with DNase on the column, washed with TR3 and 80% ethanol according to the protocol. RNA was eluted with 15-40 µl of buffer TR4.

### RNA analyses:

RNA was eluted with 15- 40 µl of buffer TR4. Spectrophotometrical analysis of purity and yield was performed on a Nanodrop photometer. RNA integrity was measured on the Agilent Bionanalyzer with a RNA 6000 Nano assay. Real time RT-PCR was performed on a TaqMan 7700 (Applied Biosystems) with a one step approach, using the Quantitect Probe RT Mix (QIAGEN) and primer and probe to amplify and detect a 294 bp fragment of the human beta actin gene.

### Example 1: RNA isolation from liver tissue mounted on a slide

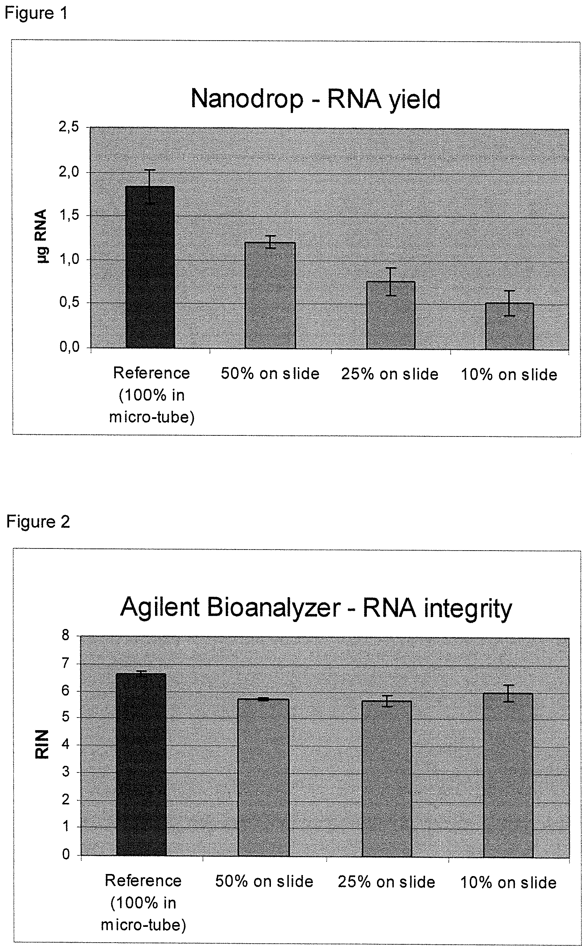

Rat liver cut into small pieces with a maximum thickness of 3 mm, was fixed for 3 h in PAXgene® Tissue Fix and 52 h in PAXgene® Tissue Stabilizer. After processing and paraffin embedding, 6 µm sections were either used for direct RNA extraction within a microcentrifuge tube according to the PAXgene® Tissue RNA kit handbook or were mounted on glass slides. Sections mounted on glass slides were deparaffinised and increasing parts of the sample (50%, 75% and 90% of the section) were removed with a cell scraper. From the remaining tissue (50%, 25%, 10% of the original section) RNA was purified using the PAXgene® Tissue RNA kit with a modified protocol for RNA extraction from sections mounted on a slide. Said protocol was modified by addition at least a part of the lysis buffer directly on top of the (remaining) sample mounted on the slide and after incubation of 10 min transferring the lysis mixture comprising the whole tissue into a microcentrifuge tube before further processing according to the kit handbook. RNA was analysed for yield by spectrophotometry on the Nanodrop® Spectrophotometer (Thermo Scientific, Wilmington, USA), for integrity on the Agilent® Bioanalyzer (Agilent Technologies, Santa Clara, USA) with a RNA 6000 Nano Assay and in a quantitative RT-PCR using a primer/probe assay for β-actin on a TaqMan 7700 (Applied Biosystems). The results of the analyses are shown in figures 1 (yield), 2 (integrity) and 3 (performance in quantitative RT-PCR).

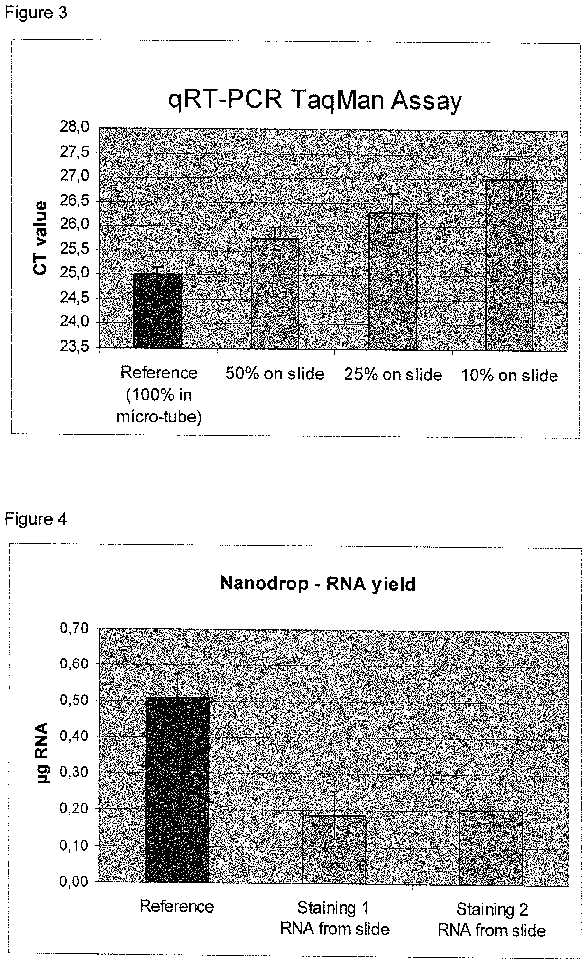

This example demonstrates that high quality RNA can be isolated from sections of PAXgene® Tissue treated samples mounted on a slide. After deparaffination, target tissue or cells can be selected, e.g. by removing unwanted areas of the section. The RNA is comparable in quality and yield to a reference, where the sections were directly transferred into a microcentrifuge tube and processed according to the PAXgene® Tissue RNA protocol. RNA can be used in demanding downstream applications like real time RT-PCR. CT values are comparable to the reference, i.e. increase in a linear way, correlating with the decreased RNA input (see Fig.3).

### Example 2: RNA isolation and histomorphology analysis from sections of tissue after staining with Hematoxylin and Eosin

Rat kidney was cut into small pieces with a maximum thickness of 4 mm and treated with the PAXgene® Tissue system. After processing and paraffin embedding triplicate samples of one 4 µm section were used for direct RNA extraction using the PAXgene® Tissue RNA kit or were mounted on glass slides and stained with Hematoxylin and Eosin. In order to avoid RNA degradation during staining the quick-staining method was used, including 60 seconds staining with Hematoxylin and 1 second staining with Eosin (Staining 1) or 30 seconds staining with Hematoxylin and 1 second staining with Eosin (Staining 2). After staining the RNA was directly isolated from the stained sections.

RNA was analysed for yield by spectrophotometry on the Nanodrop® Spectrophotometer *(Thermo Scientific, Wilmington, USA),* for integrity on the Agilent Bioanalyzer (Agilent Technologies, Santa Clara, USA) with a RNA 6000 Nano Assay and in a quantitative RT-PCR using a primer/probe assay for β-actin on a TaqMan 7700 (Applied Biosystems). Histomorphology was evaluated by staining 4µm sections with Hematoxylin and Eosin according to a standard method.

**Table 1. H&E quick-staining protocol for embedded samples**

| Step | Media | Incubation time |
|---|---|---|
| 1 | Xylene | 5 min |
| 2 | Xylene | 3 min |
| 3 | 100% Ethanol | 3 min |
| 4 | 95% Ethanol | 3 min |
| 5 | 80% Ethanol | 3 min |
| 6 | 70% Ethanol | 3 min |
| 7 | DEPC-treated water | 2 s |
| 8 | Hematoxylin solution | up to 60 s or up to 30 s |
| 9 | DEPC-treated water | 1 s |
| 10 | Eosin Y solution | 1 s |
| 11 | 80% Ethanol | 1 min |
| 12 | 95% Ethanol | 1 min |
| 13 | 100% Ethanol | Until usage |

This example demonstrates, that due to the eosinophilic properties of PFPE (PAXgene® fixed paraffin embedded) tissue sections staining for only 60 or 30 seconds with Hematoxylin and one second with Eosin is sufficient. All relevant cell structures like cytoplasma and nuclei become visible and can be used to select special cells or tissue areas for dissection. RNA in the stained sections is still preserved and can be isolated using the PAXgene® Tissue RNA kit with a modified protocol as described above. The RNA is comparable in quality and yield to a reference, where the sections was directly transferred into a microcentrifuge tube and processed according to the PAXgene® Tissue RNA protocol. RNA can be used in demanding downstream applications like real time RT-PCR and CT values are comparable to the reference.

## Claims

1. A method for determination of target cells or tissue and isolation, extraction or purification of biomolecules from a fixed biological sample, comprising the steps of:
(1) mounting at least one part of the fixed biological sample on at least one support,
(2) optionally treating at least one part of the sample with a sample treatment agent, wherein said treatment can be carried out at any stage of the procedure before step (6)
(3) staining at least one part of the fixed biological sample mounted on at least one support for not more than 60 seconds with at least one cell or tissue staining agent and rinsing the sample with water,
(4) visualizing or considering under light or backlight the at least one part stained in step 3 to determine target cells or tissue comprising biomolecules of interest,
(5) separating the target cells or tissue from the remainder of the fixed sample mounted on the support,
(6) isolating the biomolecules from the separated cells or tissue wherein the biomolecules comprise RNA;
wherein the fixed biological sample is a non-formalin-fixed sample.

2. A method according to claim 1, wherein the non-formalin-fixed biological sample is a wax embedded, preferably a paraffin embedded sample, preferably a non-formalin-fixed paraffin-embedded sample (non-FFPE-sample) and the sample treatment agent is selected from a de-waxing agent, preferably a de-paraffinizing agent selected from the group comprising toluene, xylene, linear, branched and cyclic C₆-C₁₆ alkanes, linear, branched and cyclic dialkyl ethers, wherein each alkyl chain comprises of from 6 to 16 carbon atoms, and oils liquid at room temperature (23 ± 2 °C), more preferably selected from the group comprising C₁₃-C₁₆ alkanes, dialkyl ethers of the general formula O(CₙH₂ₙ₊₁)₂, wherein n is an integer from 8 to 12, mineral oil, silicon oil and paraffin with a melting point below room temperature, preferably below room temperature, and most preferably selected from the group comprising xylene, or commericially available xylene substitutes or hexadecane, dioctylether, heptan or mixtures thereof.

3. A method according to any of claims 1 to 2, wherein the support is transparent, preferably the support is made of glass or a transparent polymer.

4. A method according to any of claims 1 to 3, wherein the sample(s) in step (4) are considered under light or backlight, preferably under a microscope.

5. A method according to any of claims 1 to 4, wherein the target cells or tissue is/are separated in step (5) by microdissection, laser capture microdissection, scraping or cutting out.

6. A method for staining non-formalin-fixed biological samples, preferably a wax-embedded non-formalin-fixed paraffin-embedded sample (non-FFPE-sample), more preferably at least one section of 0,5 to 12 µm thickness of a non-formalin-fixed paraffin-embedded sample comprising contacting said sample for not more than 60 sec, preferably less than 45 sec, more preferably less than 30 sec, most preferably less than 15 sec with at least one tissue staining agent, rinsing the sample with water and thereafter optionally contacting the sample for less than 45 sec with a further tissue staining agent.

7. The method according to claim 6, wherein the sample is contacted for up to 30 sec with Hematoxylin and less than 3 sec, preferably 1 sec with Eosin.

8. A method according to any of claims 1 to 5, wherein a non-formalin-fixed biological sample is divided in sections of 0,5 to 12 µm thickness, and
in step (1) at least two of said sections which were in the original sample adjacent to each other are mounted on separate transparent supports each,
optionally at any stage of the procedure independently from each other the sections are treated with a de-waxing agent according to step (2) in case that a wax-embedded sample was used,
in step (3) at least one of the sections is stained with at least one tissue staining agent and an adjacent section is not stained,
in step (4) cells or tissue of interest is/are determined and marked in the stained sample section and the transparent support with the unstained sample section is stacked on top of the stained and marked sample section in the same orientation as in the original sample,
in step (5) the cells or tissue of the unstained sample on top of the marked area of the stained sample is/are separated from the remainder of the unstained sample, the remainder is waived and the cells/tissue corresponding to the marked area of the stained sample is retained,
in step (6) nucleic acids are isolated or extracted from the cells retained in step (5),

9. A method according to any of claims 1 to 5, wherein a non-formalin-fixed biological sample is divided in sections of 0,5 to 12 µm thickness, and
in step (1) at least one of said sections is mounted on a transparent support,
in step (2) the section is treated with a de-waxing agent in case that a wax-embedded sample was used,
in step (3) said section is stained with at least one tissue staining agent according to the method as claimed in claim 6 or 7,
in step (4) cells or tissue of interest is/are determined in the stained sample section,
in step (5) the cells or tissue of interest is/are separated from the remainder of the stained sample, the remainder is waived,
in step (6) nucleic acids are isolated or extracted from the cells obtained in step (5).

10. Use of a non-formalin-fixed tissue stained according to a method of claim 6 or 7 for isolating biomolecules comprising RNA from at least a part thereof.

11. A method according to any of claims 1 to 5, 8 or 9 or a use according to claim 10, wherein the biomolecule isolation comprises RNA isolation.

12. Use of a kit for visualizing biomolecules comprising target cells or tissue in a non-formalin-fixed biological sample during a method according to claims 1 to 9 and 11 for extracting, isolating and/or purifying biomolecules from said target cells or tissue, wherein the kit comprises
a. optionally at least one transparent support
b. at least one tissue staining agent
c. at least one sample treatment agent as defined in claim 2
d. optionally instructions for a method according to claim 6, 7,8 or 9
e. at least one buffer and/ or equipment for isolating biomolecules, comprising RNA.

## Patentansprüche

1. Verfahren zur Bestimmung von Zielzellen oder Gewebe und zur Isolierung, Extraktion oder Reinigung von Biomolekülen aus einer fixierten biologischen Probe, umfassend die Schritte:
(1) Anbringen mindestens eines Teils der fixierten biologischen Probe auf mindestens einem Träger,
(2) gegebenenfalls Behandeln von wenigstens einem Teil der Probe mit einem Probenbehandlungsmittel, wobei die Behandlung in jedem Stadium des Verfahrens vor dem Schritt (6) durchgeführt werden kann,
(3) Färben wenigstens eines Teils der fixierten biologischen Probe, die auf dem mindestens einen Träger angebracht ist, für nicht mehr als 60 Sekunden mit wenigstens einem Zell- oder Gewebefärbemittel und Spülen der Probe mit Wasser,
(4) Sichtbarmachen oder Betrachten unter Licht oder bei Hintergrundsbeleuchtung des wenigstens einen Teils, der in Schritt 3 gefärbt wurde, um Zielzellen oder Gewebe, die/das Biomoleküle von Interesse enthalten, zu bestimmen,
(5) Abtrennen der Zielzellen oder -gewebes von dem Rest der auf dem Träger angebrachten Probe;
(6) Isolieren der Biomoleküle aus den abgetrennten Zellen oder dem Gewebe, wobei die Biomoleküle RNA umfassen,
wobei die fixierte biologische Probe eine nicht mit Formalin fixierte biologische Probe ist.

2. Verfahren nach Anspruch 1, wobei die nicht mit Formalin fixierte biologische Probe eine in Wachs eingebettete, vorzugsweise eine in Paraffin eingebettete Probe ist, vorzugsweise eine nicht mit Formalin fixierte, in Paraffin eingebettete Probe (Nicht-FFPE-Probe) und das Probenbehandlungsmittel ausgewählt ist aus einem entwachsenden Mittel, vorzugsweise einem Entparaffinierungsmittel, ausgewählt aus der Gruppe umfassend Toluol, Xylol, lineare, verzweigte und zyklische C₆-C₁₆-Alkane, lineare, verzweigte und zyklische Dialkylether, wobei jede Alkylkette 6 bis 16 Kohlenstoffatome umfasst und Öle, die bei Raumtemperatur (23 ± 2 ° C) flüssig sind, besonders bevorzugt ausgewählt aus der Gruppe umfassend C₁₃-C₁₆-Alkane, Dialkylether der allgemeinen Formel O(CₙH₂ₙ₊₁)₂, worin n eine ganze Zahl von 8 bis 12 ist, Mineralöl, Silikonöl und Paraffin mit einem Schmelzpunkt unterhalb von Raumtemperatur, bevorzugt unter Raumtemperatur, und am meisten bevorzugt ausgewählt aus der Gruppe enthaltend Xylol oder kommerziell erhältliche Xylol-Ersatzstoffe oder Hexadecan, Dioctylether, Heptan oder Mischungen davon.

3. Verfahren nach einem der Ansprüche 1 oder 2, wobei der Träger transparent ist, vorzugsweise der Träger aus Glas oder einem transparenten Polymer hergestellt ist.

4. Verfahren nach einem der Ansprüche 1 bis 3, wobei die Probe(n) in Schritt (4) unter Licht oder Hintergrundbeleuchtung, vorzugsweise unter einem Mikroskop, betrachtet werden.

5. Verfahren nach einem der Ansprüche 1 bis 4, wobei die Zielzellen oder das Zielgewebe in Schritt (5) durch Mikrodissektion, Laser Capture Mikrodissektion, Kratzen oder Ausschneiden abgetrennt werden.

6. Ein Verfahren zum Anfärben von nicht mit Formalin fixierten biologischen Proben, bevorzugt einer in Wachs eingebetteten, nicht mit Formalin fixierten, in Paraffin eingebetteten Probe (Nicht-FFPE-Probe), weiter bevorzugt wenigstens eines Schnitts von 0,5 bis 12 µm Dicke einer nicht mit Formalin fixierten, in Paraffin eingebetteten Probe, umfassend das in-Kontakt-Bringen der Probe für nicht mehr als 60 Sekunden, bevorzugt für weniger als 45 Sekunden, weiter bevorzugt für weniger als 30 Sekunden, am meisten bevorzugt für weniger als 15 Sekunden mit wenigstens einem Gewebe-Färbemittel, Spülen der Probe mit Wasser und anschließend gegebenenfalls das Kontaktieren der Probe für weniger als 45 Sekunden mit einem weiteren Gewebefärbemittel.

7. Verfahren nach Anspruch 6, wobei die Probe für bis zu 30 Sekunden mit Hämatoxylin und für weniger als 3 Sekunden, vorzugsweise 1 Sekunde mit Eosin in Kontakt gebracht wird.

8. Verfahren nach einem der Ansprüche 1 bis 5, wobei eine nicht mit Formalin fixierte biologische Probe in Schnitte von 0,5 bis 12 µm Dicke geteilt wird, und in Schritt (1) wenigstens zwei dieser Schnitte, die in der ursprünglichen Probe zueinander benachbart waren, jeweils auf separaten transparenten Trägern angebracht werden,
gegebenenfalls in jedwelchem Stadium des Verfahrens unabhängig voneinander die Schnitte mit einem entwachsenden Mittel gemäß Schritt (2) behandelt werden, falls eine in Wachs eingebettete Probe verwendet wurde,
in Schritt (3) wenigstens einer der Schnitte mit mindestens einem Gewebefärbemittel gefärbt und ein benachbarter Schnitt nicht gefärbt wird,
in Schritt (4) die Zellen oder das Gewebe von Interesse in dem gefärbten Probenabschnitt bestimmt und markiert werden/wird und der transparente Träger mit dem ungefärbten Probenschnitt auf den gefärbten und markierten Probenschnitt in der gleichen Ausrichtung wie in der ursprünglichen Probe aufgelegt wird,
in Schritt (5) die Zellen oder das Gewebe der ungefärbten Probe oberhalb des markierten Bereichs der gefärbten Probe von dem Rest der ungefärbten Probe abgetrennt werden, der Rest verworfen wird und die dem markierten Bereich der gefärbten Probe entsprechenden Zellen / Gewebe behalten werden,
in Schritt (6) die Nukleinsäuren aus den in Schritt (5) behaltenen Zellen isoliert oder extrahiert werden.

9. Verfahren nach einem der Ansprüche 1 bis 5, wobei eine nicht mit Formalin fixierte biologische Probe in Schnitte von 0,5 bis 12 µm Dicke unterteilt wird und in Schritt (1) mindestens einer der Schnitte auf einem transparenten Träger angebracht wird,
in Schritt (2) der Schnitt mit einem entwachsenden Mittel behandelt wird, falls eine in Wachs eingebettete Probe verwendet wurde,
in Schritt (3) der Schnitt mit mindestens einem Gewebefärbemittel gemäß dem Verfahren nach Anspruch 6 oder 7 gefärbt wird,
in Schritt (4) Zellen oder Gewebe von Interesse in dem gefärbten Probenschnitt bestimmt werden/wird,
in Schritt (5) die Zellen oder das Gewebe von Interesse von dem Rest der gefärbten Probe getrennt werden/wird, wobei der Rest verworfen wird,
in Schritt (6) Nukleinsäuren aus den in Schritt (5) erhaltenen Zellen isoliert oder extrahiert werden.

10. Verwendung eines nach einem Verfahren gemäß Anspruch 6 oder 7 gefärbten Gewebes zum Isolieren von Biomolekülen, die RNA umfassen, aus mindestens einem Teil davon.

11. Verfahren nach einem der Ansprüche 1 bis 5, 8 oder 9 oder Verwendung nach Anspruch 10, wobei die Biomolekülisolierung eine RNA-Isolierung umfasst.

12. Verwendung eines Kits zum Sichtbarmachen von Biomolekülen, die Zielzellen oder -gewebe in einer nicht mit Formalin fixierten biologischen Probe umfassen, während eines Verfahrens nach den Ansprüchen 1 bis 9 und 11 zum Extrahieren, Isolieren und / oder Aufreinigen von Biomolekülen aus den Zielzellen oder dem Zielgewebe, wobei der Kit enthält:
a. gegebenenfalls wenigstens einen transparenten Träger
b. mindestens ein Gewebefärbemittel
c. wenigstens ein Probenbehandlungsmittel wie in Anspruch 3 definiert,
d. gegebenenfalls Anweisungen für ein Verfahren nach Anspruch 6, 7, 8 oder 9
e. wenigstens einen Puffer und / oder Gerätschaften zum Isolieren von Biomolekülen, die RNA umfassen.

## Revendications

1. Procédé pour la détermination de cellules cibles ou de tissu cible et l'isolement, l'extraction ou la purification de biomolécules à partir d'un échantillon biologique fixé, comprenant les étapes consistant à :
(1) monter sur au moins un support au moins une partie de l'échantillon biologique fixé,
(2) en option traiter au moins une partie de l'échantillon par un agent de traitement d'échantillon, ledit traitement pouvant être effectué à un stade quelconque de la procédure avant l'étape (6),
(3) colorer au moins une partie de l'échantillon biologique fixé, monté sur au moins un support, pendant une durée n'excédant pas 60 secondes, à l'aide d'au moins un agent de coloration de cellules ou de tissus, et rincer avec de l'eau l'échantillon,
(4) visualiser ou examiner sous éclairage ou rétro-éclairage ladite au moins une partie colorée dans l'étape 3 pour déterminer les cellules cibles ou le tissu cible comprenant des biomolécules d'intérêt,
(5) séparer les cellules cibles ou le tissu cible du reste de l'échantillon fixé, monté sur le support,
(6) isoler les biomolécules des cellules séparées ou du tissu séparé, les biomolécules comprenant de l'ARN ;
l'échantillon biologique fixé étant un échantillon non fixé au formol.

2. Procédé selon la revendication 1, dans lequel l'échantillon biologique non fixé au formol est un échantillon inclus dans de la cire, de préférence inclus dans de la paraffine, de préférence un échantillon non fixé au formol et inclus dans de la paraffine (échantillon non-FFPE) et l'agent de traitement d'échantillon est choisi parmi un agent de décirage, de préférence un agent de déparaffinage choisi dans l'ensemble comprenant le toluène, le xylène, des alcanes en C₆-C₁₆ linéaires, ramifiés ou cycliques, des éthers dialkyliques linéaires, ramifiés ou cycliques, dans lesquels chaque chaîne alkyle comprend de 6 à 16 atomes de carbone, et des huiles liquides à la température ambiante (23 ± 2 °C), de façon plus particulièrement préférée choisi dans l'ensemble comprenant des alcanes en C₁₃-C₁₆, des éthers dialkyliques de formule générale O(CₙH₂ₙ₊₁)₂, dans laquelle n est un nombre entier valant de 8 à 12, l'huile minérale, l'huile de silicone et une paraffine ayant un point de fusion inférieur à la température ambiante, de préférence inférieur à la température ambiante, et de façon tout particulièrement préférée choisi dans l'ensemble comprenant le xylène, ou des substituts de xylène disponibles dans le commerce ou l'hexadécane, l'éther dioctylique, l'heptane ou des mélanges de ceux-ci.

3. Procédé selon l'une quelconque des revendications 1 et 2, dans lequel le support est transparent, de préférence le support et fait de verre ou d'un polymère transparent.

4. Procédé selon l'une quelconque des revendications 1 à 3, dans lequel l'échantillon/les échantillons dans l'étape (4) est(sont) examiné(s) sous éclairage ou rétro-éclairage, de préférence sous un microscope.

5. Procédé selon l'une quelconque des revendications 1 à 4, dans lequel les cellules cibles ou le tissu cible sont/est séparé(es) dans l'étape (5) par microdissection, microdissection par capture laser, raclage ou excision.

6. Procédé pour la coloration d'échantillons biologiques non fixés au formol, de préférence d'un échantillon inclus dans de la cire, non fixé au formol et inclus dans de la paraffine (échantillon non-FFPE), de façon plus particulièrement préférée d'au moins une coupe de 0,5 à 12 µm d'épaisseur d'un échantillon non fixé au formol et inclus dans de la paraffine, comprenant la mise en contact dudit échantillon pendant plus une durée n'excédant pas 60 secondes, de préférence inférieure à 45 secondes, de façon plus particulièrement préférée inférieure à 30 secondes, de façon tout particulièrement préférée inférieure à 15 secondes, avec au moins un agent de coloration de tissus, le rinçage de l'échantillon avec de l'eau et ensuite en option la mise en contact de l'échantillon pendant moins de 45 secondes avec un autre agent de coloration de tissus.

7. Procédé selon la revendication 6, dans lequel l'échantillon est mis en contact pendant jusqu'à 30 secondes avec de l'hématoxyline et pendant moins de 3 secondes, de préférence 1 seconde avec de l'éosine.

8. Procédé selon l'une quelconque des revendications 1 à 5, dans lequel on divise un échantillon biologique non fixé au formol en coupes de 0,5 à 12 µm d'épaisseur, et
dans l'étape (1) on monte sur des supports transparents distincts au moins deux desdites coupes qui dans l'échantillon initial étaient adjacentes entre elles,
en option à un stade quelconque du processus on traite indépendamment l'une de l'autre (les unes des autres) les coupes par un agent de décirage selon l'étape (2) dans le cas où un échantillon inclus dans de la cire a été utilisé,
dans l'étape (3) au moins une des coupes est colorée avec au moins un agent de coloration de tissus et une coupe adjacente n'est pas colorée,
dans l'étape (4) on détermine des cellules ou un tissu d'intérêt et on le(s) marque dans la coupe d'échantillon colorée et on empile le support transparent avec la coupe d'échantillon non colorée au-dessus de la coupe d'échantillon colorée et marquée, dans la même orientation que dans l'échantillon initial,
dans l'étape (5) on sépare du reste de l'échantillon non coloré les cellules ou le tissu de l'échantillon non coloré au-dessus de la zone marquée de l'échantillon coloré, on rejette le reste et on conserve les cellules/le tissu correspondant à la zone marquée de l'échantillon coloré,
dans l'étape (6) on isole ou extrait les acides nucléiques des cellules conservées dans l'étape (5).

9. Procédé selon l'une quelconque des revendications 1 à 5, dans lequel on divise un échantillon biologique non fixé au formol en coupes de 0,5 à 12 µm d'épaisseur, et
dans l'étape (1) on monte sur un support transparent au moins une desdits coupes, dans l'étape (2) on traite la coupe par un agent de décirage dans le cas où un échantillon inclus dans de la cire a été utilisé,
dans l'étape (3) on colore ladite coupe avec au moins un agent de coloration de tissus selon le procédé tel que revendiqué dans la revendication 6 ou 7,
dans l'étape (4) on détermine les cellules ou le tissu d'intérêt dans la coupe d'échantillon colorée,
dans l'étape (5) on sépare du reste de l'échantillon coloré les cellules ou le tissu d'intérêt, on rejette le reste,
dans l'étape (6) on isole ou extrait les acides nucléiques des cellules obtenues dans l'étape (5).

10. Utilisation d'un tissu non fixé au formol, coloré selon un procédé de la revendication 6 ou 7, pour l'isolement de biomolécules comprenant de l'ARN d'avec au moins une partie de celles-ci.

11. Procédé selon l'une quelconque des revendications 1 à 5, 8 ou 9, ou utilisation selon la revendication 10, dans lequel l'isolement de biomolécules comprend un isolement d'ARN.

12. Utilisation d'un nécessaire pour la visualisation de cellules cibles ou de tissu cible comprenant des biomolécules dans un échantillon biologique non fixé au formol, au cours d'un procédé selon les revendications 1 à 9 et 11 pour l'extraction, l'isolement et/ou la purification de biomolécules à partir desdites cellules cibles ou dudit tissu cible, dans laquelle le nécessaire comprend
a. en option au moins un support transparent
b. au moins un agent de coloration de tissus
c. au moins un agent de traitement d'échantillon tel que défini dans la revendication 2
d. en option des directives pour un procédé selon la revendication 6, 7, 8 ou 9
e. au moins un tampon et/ou appareillage pour l'isolement de biomolécules, comprenant de l'ARN.
